(19)

Europäisches
Patentamt
European
Patent Office
Office européen
des brevets

(11)  EP 4 345 830 A1

(12)  EUROPEAN PATENT APPLICATION

(43) Date of publication:
03.04.2024   Bulletin 2024/14

(21) Application number: 23200125.5

(22) Date of filing: 27.09.2023

(51) International Patent Classification (IPC):
G16H 20/40 (2018.01)        A61B 34/10 (2016.01)
G16H 40/63 (2018.01)

(52) Cooperative Patent Classification (CPC):
G16H 20/40; A61B 34/10; G06N 3/02; G16H 40/63;
A61B 2018/00529; A61B 2018/00577;
A61B 2018/00642; A61B 2034/105; A61B 2034/107

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(30) Priority: 28.09.2022   US 202217935945
28.09.2022   EP 22465554

(71) Applicant: Siemens Healthineers AG
91301 Forchheim (DE)

(72) Inventors:
• CHAITANYA, Krishna
8051 Zürich (CH)
• AUDIGIER, Chloé
3006 Bern (CH)
• PAILLARD, Joseph
35700 Rennes (FR)
• BALASCUTA, Laura Elena
507165 Prejmer, Brasov (RO)
• GHESU, Florin-Cristian
91083 Baiersdorf (DE)
• COMANICIU, Dorin
Princeton, NJ, 08540 (US)
• MANSI, Tommaso
Plainsboro, NJ, 08536 (US)

(74) Representative: Horn Kleimann Waitzhofer
Schmid-Dreyer
Patent- und Rechtsanwälte PartG mbB
Theresienhöhe 12
80339 München (DE)

(54) **AUTOMATIC PLANNING AND GUIDANCE OF LIVER TUMOR THERMAL ABLATION USING AI AGENTS TRAINED WITH DEEP REINFORCEMENT LEARNING**

(57)    Systems and methods for determining an optimal position of one or more ablation electrodes are provided. A current state of an environment is defined based on a mask of one or more anatomical objects and one or more current positions of one or more ablation electrodes. The one or more anatomical objects comprise one or more tumors. For each particular AI (artificial intelligence) agent of one or more AI agents, one or more actions for updating the one or more current positions of a respective ablation electrode of the one or more ablation electrodes in the environment are determined based on the current state using the particular AI agent. A next state of the environment is defined based on the mask and the one or more updated positions of the respective ablation electrode. The steps of determining the one or more actions and defining the next state are repeated for a plurality of iterations using 1) the next state as the current state and 2) the one or more updated positions as the one or more current positions to determine one or more final positions of the respective ablation electrode for performing a thermal ablation on the one or more tumors. The one or more final positions of each respective ablation electrode are output.

FIG 1

100
Receive one or more input medical images of a patient
102

Generate a mask of one or more anatomical objects from the one or more input medical images
104

Define a current state of an environment based on the mask and one or more current positions of one or more ablation electrodes
106

Determine one or more actions for updating the one or more current positions of a respective ablation electrode of the one or more ablation electrodes in the environment based on the current state using the particular AI agent
108

Define a next state of the environment based on the mask and the one or more updated positions of the respective ablation electrode
110

Repeat the steps of determining the one or more actions and defining the next state for a plurality of iterations to iteratively update the one or more current positions of the respective ablation electrode using 1) the next state as the current state and 2) the one or more updated positions as the one or more current positions to determine one or more final positions of the respective ablation electrode for performing an ablation on the one or more tumors
112

Output the one or more final positions of each respective ablation electrode
114

**Description**

TECHNICAL FIELD

The present invention relates generally to automatic planning and guidance of liver tumor thermal ablation, and in particular to automatic planning and guidance of liver tumor thermal ablation using AI (artificial intelligence) agents trained with deep reinforcement learning.

BACKGROUND

Thermal ablation refers to the destruction of tissue by extreme hyperthermia and is a minimally invasive alternative to resection and transplantation for the treatment of liver tumors. Thermal ablation of liver cancer has emerged as a first-line curative treatment for tumors as thermal ablation has similar overall survival rates as surgical resection but is far less invasive, has lower complication rates, has superior cost-effectiveness, and has an extremely low treatment-associated mortality.

Planning of thermal ablation is typically performed manually by clinicians visualizing CT (computed tomography) images in 2D. However, such manual planning of thermal ablation is time consuming, challenging, and can lead to incomplete tumor ablation. Recently, conventional approaches have been proposed for the automatic planning of thermal ablations. However, such conventional approaches are computationally expensive with a high inference time per patient.

BRIEF SUMMARY OF THE INVENTION

In accordance with one or more embodiments, systems and methods for determining an optimal position of one or more ablation electrodes for automatic planning and guidance of tumor thermal ablation are provided. A current state of an environment is defined based on a mask of one or more anatomical objects and one or more current positions of one or more ablation electrodes. The one or more anatomical objects comprise one or more tumors. For each AI (artificial intelligence) agent of one or more AI agents, one or more actions for updating the one or more current positions of a respective ablation electrode of the one or more ablation electrodes in the environment are determined based on the current state using the particular AI agent. A next state of the environment is defined based on the mask and the one or more updated positions of the respective ablation electrode. The steps of determining the one or more actions and defining the next state are repeated for a plurality of iterations to iteratively update the one or more current positions of the respective ablation electrode using 1) the next state as the current state and 2) the one or more updated positions as the one or more current positions to determine one or more final positions of the respective ablation electrode for performing a thermal ablation on the one or more tumors. The one or more final positions of each respective ablation electrode are output.

In one embodiment, the one or more current positions of one or more ablation electrodes comprise one or more of an electrode tumor endpoint or an electrode skin endpoint. The one or more anatomical objects may further comprise one or more organs and skin of a patient.

In one embodiment, defining the next state of the environment comprises updating a net cumulative reward for the particular AI agent, where the net cumulative reward is defined based on clinical constraints. The steps of determining the one or more actions and defining the next state are repeated until the net cumulative reward satisfies a threshold value.

In one embodiment, one or more discrete predefined actions are determined using the particular AI agent implemented using a double deep Q network. In another embodiment, a continuous action is determined using the particular AI agent implemented using proximal policy optimization.

In one embodiment, the current state of the environment is defined based on an ablation zone of each of the plurality of ablation electrodes. The ablation zones are modeled as an ellipsoid.

In one embodiment, the one or more AI agents may comprise a plurality of AI agents. The one or more actions are determined based on the same current state for the plurality of AI agents and/or based on a joint net cumulative reward for the plurality of AI agents.

In one embodiment, intraoperative guidance for performing a thermal ablation on the one or more tumors is generated based on the one or more final positions.

In one embodiment, the one or more AI agents comprises a plurality of AI agents trained according to different ablation parameters. Optimal ablation parameters for performing an ablation on the one or more tumors is determined by selecting at least one of the plurality of AI agents.

In accordance with one or more embodiments, systems and methods for determining an optimal position of a plurality of ablation electrodes are provided. A current state of an environment is defined based on a mask of one or more anatomical objects, a current position of an electrode tumor endpoint of each of a plurality of ablation electrodes, and a current position of an electrode skin endpoint of each of the plurality of ablation electrodes. The one or more anatomical objects comprise one or more tumors. For each particular AI (artificial intelligence) agent of a plurality of AI agents, one or more actions for updating the current position of the electrode tumor endpoint of a respective ablation electrode of the plurality of ablation electrodes and the current position of the electrode skin endpoint of the respective ablation electrode in the environment are determined based on the current state using the particular AI agent. A next state of the environment is defined based on the mask, the updated position of the electrode tumor endpoint of the respective ablation electrode, and the updated position of the electrode skin endpoint of the respective ablation electrode. The steps of determining the one or more actions and defining the next state are repeated for a plurality of iterations to iteratively update the current position of the electrode tumor endpoint and the current position of the electrode skin endpoint using 1) the next state as the current state, 2) the updated position of the electrode tumor endpoint of the respective ablation electrode as the current position of the electrode tumor endpoint of the respective ablation electrode, and 3) the updated position of the electrode skin endpoint of the respective ablation electrode as the current position of the electrode skin endpoint of the respective ablation electrode to determine a final position of the electrode tumor endpoint of the respective ablation electrode and a final position of the electrode skin endpoint of the respective ablation electrode for performing a thermal ablation on the one or more tumors. The final position of the electrode tumor endpoint and the final position of the electrode skin endpoint of each of the plurality of ablation electrodes are output.

In one embodiment, defining a next state of the environment comprises updating a joint net cumulative reward for the plurality of AI agents, where the joint net cumulative reward is defined based on clinical constraints. The steps of determining the one or more actions and defining the next state are repeated until the joint net cumulative reward satisfies a threshold value.

These and other advantages of the invention will be apparent to those of ordinary skill in the art by reference to the following detailed description and the accompanying drawings.

BRIEF DESCRIPTION OF THE DRAWINGS

Figure 1 shows a method for determining a position of one or more ablation electrodes for performing a thermal ablation on one or more tumors, in accordance with one or more embodiments;

Figure 2 shows a workflow for determining a position of one or more ablation electrodes for performing a thermal ablation on one or more tumors, in accordance with one or more embodiments;

Figure 3 shows an environment in which a current state $S_t$ is defined, in accordance with one or more embodiments;

Figure 4 shows a framework for determining a discrete set of predefined actions using a DDΓaN, in accordance with one or more embodiments;

Figure 5 shows a framework for determining a continuous action using PPO (proximal policy optimization), in accordance with one or more embodiments;

Figure 6 shows a method for respectively determining a position of a plurality of ablation electrodes for performing a thermal ablation on one or more tumors, in accordance with one or more embodiments;

Figure 7 shows a visualization of an action space of an ablation electrode, in accordance with one or more embodiments;

Figure 8 shows a workflow for training a plurality of AI agents for respectively determining a position of a plurality of ablation electrodes for performing a thermal ablation on one or more tumors using MARL (multi-agent reinforcement learning), in accordance with one or more embodiments;

Figure 9 shows an exemplary artificial neural network that may be used to implement one or more embodiments;

Figure 10 shows a convolutional neural network that may be used to implement one or more embodiments; and

Figure 11 shows a high-level block diagram of a computer that may be used to implement one or more embodiments.

DETAILED DESCRIPTION

The present invention generally relates to methods and systems for automatic planning and guidance of liver tumor thermal ablation using AI (artificial intelligence) agents trained with deep reinforcement learning. Embodiments of the present invention are described herein to give a visual understanding of such methods and systems. A digital image is often composed of digital representations of one or more objects (or shapes). The digital representation of an object is often described herein in terms of identifying and manipulating the objects. Such manipulations are virtual manipulations accomplished in the memory or other circuitry/hardware of a computer system. Accordingly, is to be understood that embodiments of the present invention may be performed within a computer system using data stored within the computer system.

Embodiments described herein provide for a DRL (deep reinforcement learning) approach for determining an optimal position of one or more ablation electrodes for performing thermal ablation that satisfies all clinical constraints and does not require any labels in training. DRL is a framework where AI agents, represented by a machine learning based networks (e.g., neural networks), learn how to iteratively displace or update a current position of the ablation electrode within a custom environment to move from a current state to a terminal state of the custom environment. The current state is iteratively updated based on the AI agent's action in updating the position of the ablation electrode. The objective is to maximize a net cumulative reward by learning an optimal policy that gives a set of actions to determine the optimal position of the ablation electrode. Advantageously, embodiments described herein enable automatic planning and guidance during thermal ablation procedures with low inference time and without any manual annotations required during training to achieve 100% tumor coverage while satisfying all clinical constraints.

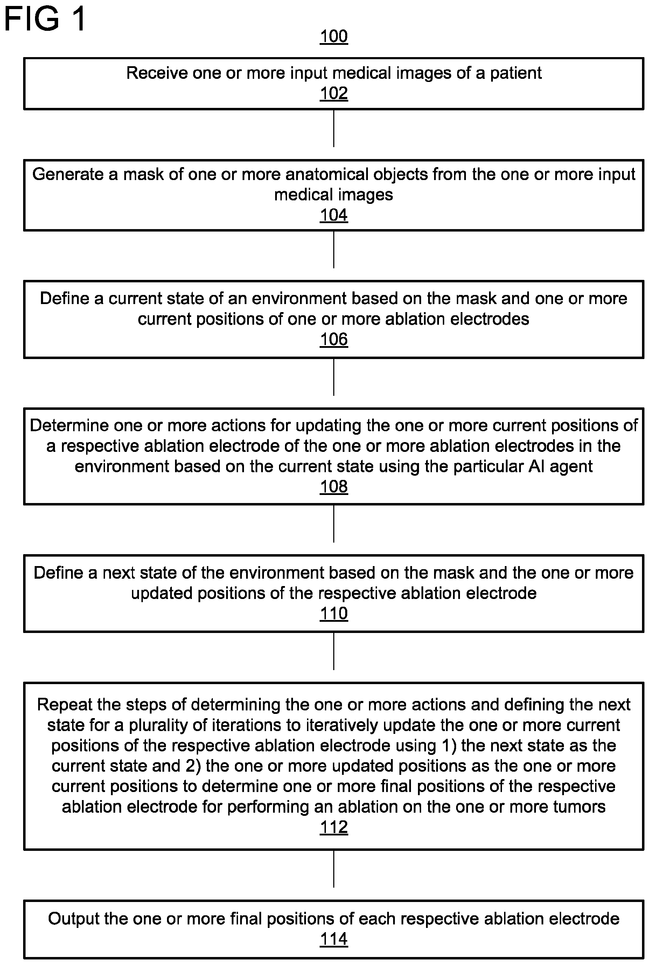

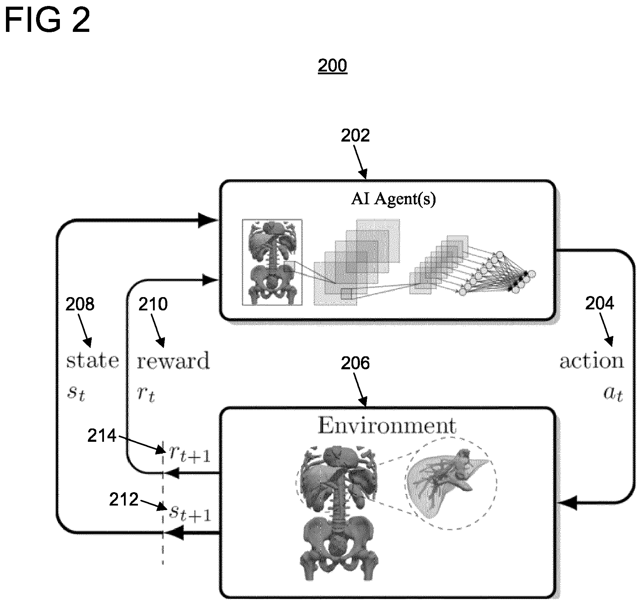

Figure 1 shows a method 100 for determining a position of one or more ablation electrodes for performing a thermal ablation on one or more tumors, in accordance with one or more embodiments. The steps of method 100 may be performed by one or more suitable computing devices, such as, e.g., computer 1102 of Figure 110. Figure 2 shows a workflow 200 for determining a position of one or more ablation electrodes for performing a thermal ablation on one or more tumors, in accordance with one or more embodiments. Figure 1 and Figure 2 will be described together.

At step 102 of Figure 1, one or more input medical images of a patient are received. The input medical images depict one or more tumors on which thermal ablation will be performed in accordance with method 100. In one embodiment, the input medical images depict the one or more tumors on a liver of the patient. However, the input medical images may depict the one or more tumors on any other suitable anatomical structures of interest of the patient (e.g., other organs, bones, etc.).

In one embodiment, the input medical images are CT images. However, the input medical images may comprise any other suitable modality, such as, e.g., MRI (magnetic resonance imaging), ultrasound, x-ray, or any other medical imaging modality or combinations of medical imaging modalities. In one embodiment, the input medical images comprise at least one 3D (three dimensional) volume. However, the input medical images may additionally comprise at least one 2D (two dimensional) image, and may comprise a single input medical image or a plurality of input medical images. The input medical images may be received directly from an image acquisition device, such as, e.g., a CT scanner, as the medical images are acquired, or can be received by loading previously acquired medical images from a storage or memory of a computer system or receiving medical images that have been transmitted from a remote computer system.

At step 104 of Figure 1, a mask of one or more anatomical objects is generated from the one or more input medical images. The anatomical objects comprise the one or more tumors. In one embodiment, the anatomical objects may also comprise organs at risk (OAR), skin of the patient, or any other suitable anatomic objects of interest (e.g., blood vessels). The organs at risk refer to organs in the vicinity of the one or more tumors in the input medical images.

The mask may be generated using any suitable approach. In one embodiment, the mask comprises one or more segmentation masks of the anatomical objects generated by automatically segmenting the anatomical objects from the input medical images. The segmentation may be performed using any suitable approach.

At step 106 of Figure 1, a current state $S_t$ of an environment is defined based on the mask m and one or more current positions of the respective ablation electrode. In one example, as shown in Figure 2, the current state $S_t$ is state $S_t$ 208 of environment 206.

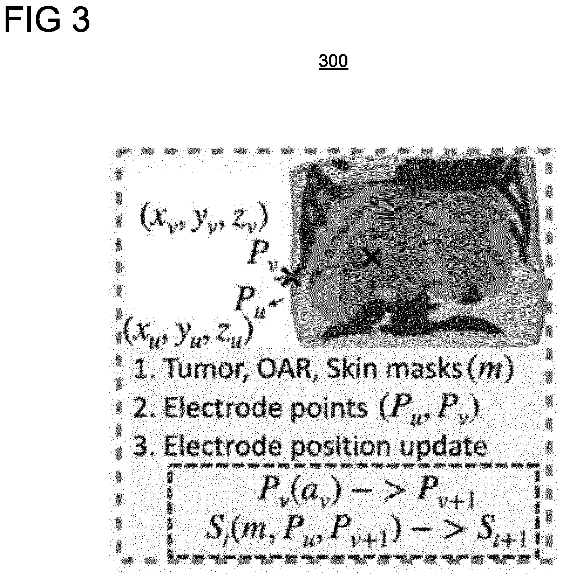

Figure 3 shows an environment 300 in which a current state $S_t$ is defined, in accordance with one or more embodiments. Environment 300 comprises 3D information of the input medical image, the mask m and one or more current positions of the respective ablation electrode. As shown in Figure 3, the current state $S_t$ is defined based on the mask m of the one or more anatomical objects (e.g., tumors, organs at risk, and skin) and the one or more current positions of the respective ablation electrode. In one embodiment, the current positions of the respective ablation electrode comprise one or more of an electrode tumor endpoint $P_u = (x_u, y_u, z_u)$ or an electrode skin endpoint $P_v = (x_v, y_v, z_v)$. The current state $S_t$ is therefore defined as $S_t = (m, P_u, P_v)$.

The electrode tumor endpoint $P_u$ represents the current position of the tip of the respective ablation electrode. In method 100 of Figure 1, in accordance with one embodiment, the position of the electrode tumor endpoint $P_u$ will be assumed to be fixed at the center of the tumor. In this embodiment, the ablation zone is modeled as a sphere centered at electrode tumor endpoint $P_u$ with a radius selected from a given set of valid radius values to achieve 100% tumor coverage. However, in other embodiments, the ablation zone may be modeled using any other suitable shape (e.g., ellipsoid) and thus the electrode tumor endpoint $P_u$ may be optimized at any location and is not necessarily fixed at the center of the tumor.

The electrode skin endpoint $P_v$ represents the position on the respective ablation electrode that intersects the skin of the patient. The electrode skin endpoint $P_v = (x_v, y_v, z_v)$ may be initially randomly assigned outside the skin surface while ensuring an electrode length of less than, e.g., 150 mm (millimeters), which may be defined as a clinical constraint.

Steps 108-112 of Figure 1 are performed for each particular AI agent of the one or more AI agents. Each particular AI agent iteratively updates the one or more positions of a respective ablation electrode for performing a thermal ablation on one or more tumors.

At step 108 of Figure 1, one or more actions $a_t$ for updating the one or more current positions of the respective ablation electrode in the environment are determined based on the current state using the particular AI agent. The one or more actions $a_t$ are determined based on a net cumulative reward $r_t$ defined based on clinical constraints. The objective is to maximize the net cumulative reward $r_t$ by learning an optimal policy that gives a set of actions $a_t$ for updating the current positions of the respective ablation electrode to reach a terminal state from the current state. As electrode tumor endpoint $P_u$ is assumed to be fixed in method 100, the one or more actions $a_t$ update or displace the current position of electrode skin endpoint $P_v$ in the environment to an updated position of electrode skin endpoint $P_{v+1}$ in the environment.

In one example, as shown in Figure 2, AI agent 202 determines actions $a_t$ 204 for updating the current positions of the respective ablation electrode in environment 206 based on the current state $S_t$ 208 and the net cumulative reward $r_t$ 210. Based on the updated positions of the respective ablation electrode in environment 206, the current state $S_t$ 208 is updated to updated state $S_{t+1}$ 212 and the net cumulative reward $r_t$ 210 is updated to $r_{t+1}$ 214.

The net cumulative reward $r_t$ is defined according to clinical constraints. Table 1 shows exemplary rewards for updating the current position of electrode skin endpoint $P_v$, in accordance with one or more embodiments.

Table 1: Table of clinical constraints and corresponding rewards for electrode skin endpoint $P_v$. For constraint 3, $r_l$ is positive when the electrode length is less than 150 mm and negative when $r_l$ is greater than 150 mm.

| Clinical Constraints | | Rewards for electrode skin endpoint $P_v$ |
|---|---|---|
| 1. | Electrode trajectory must not collide with organs at risk or with hepatic vessels | +1 |
| 2. | Electrode skin endpoint $P_v$ must be outside the body of the patient | +1 |
| 3. | Length $l_e$ of the ablation electrode within the body of the patient is less than the maximum allowed electrode length (e.g., 150 mm) | $r_l = (150 - l_e)/150$ |
| 4. | The distance $d_{OAR}$ between organs at risk and the ablation electrode is at least, e.g., 12 mm | $r_d = d_{OAR}/100$ |
| 5. | The ablation zone at electrode tumor endpoint $P_u$ must have 100% tumor coverage | N/A |
| 6. | The ablation zone must not have any collisions with organs at risk | N/A |

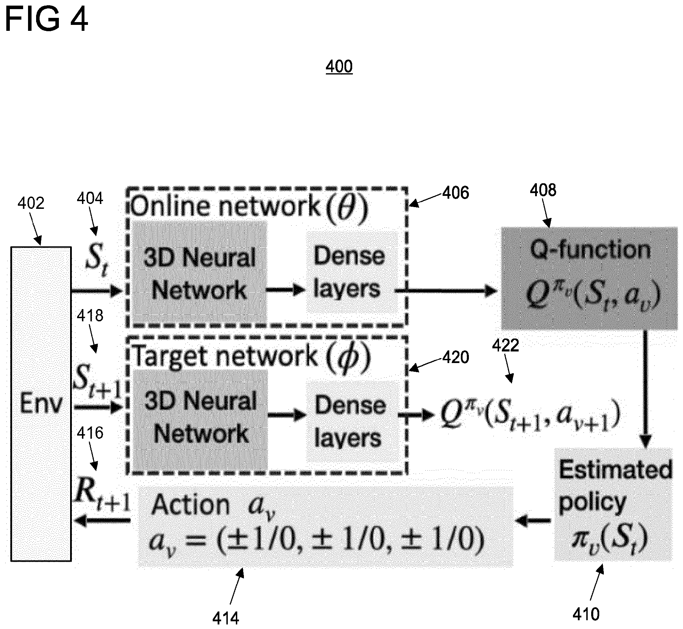

In one embodiment, the one or more actions comprise a set of discrete predefined actions modeled using a value learning approach with the AI agents implemented using a DDQN (double deep Q network). Figure 4 shows a framework 400 for determining a discrete set of predefined actions using a DDΓaN, in accordance with one or more embodiments. Given a current state $S_t$ 404 observed in environment 402, online network 406, comprising a 3D neural network and dense layers, estimates Q-values $Q^{\pi v}(S_t, a_v)$ 408 for all predefined actions $a_v$. The action with the highest Q-value is used to estimate action policy $\pi_v(S_t)$ 410 to select the best action $a_v$ 414 for updating the current position of electrode skin endpoint $P_v$ in environment 402. Based on selected action $a_v$ 414, an updated state $S_{t+1}$ 418 and updated net cumulative reward $r_{t+1}$ 416 are determined. The discrete action values are $a_v = (\pm 1/0, \pm 1/0, \pm 1/0)$, indicating that each coordinate of the electrode skin endpoint $P_v$ can be updated by +1, -1, or 0 (i.e., no displacement) in the 3D space of environment 402. Accordingly, the dense layers of online network 406 output 27(3x3) Q-values corresponding to the combination of all possible actions. The electrode skin endpoint $P_v$ is updated to $P_{v+1} = P_v(a_v) = (x_v \pm 1/0, y_v \pm 1/0, z_v$

± 1/0). The target network 420, comprising a 3D neural network and dense layers, estimates updated Q-values $Q^{\pi v}(S_{t+1}, a_{v+1})$ 422 from updated state $S_{t+1}$ 418.

As shown in Figure 4, framework 400 comprises two networks: online network 406 parameterized by weights $\theta$ and target network 420 parameterized by weights $\phi$. Target network 420 estimates an optimal Q-value function during a training stage. Online network 406 chooses a best action given the estimated Q-value function from target network 420 and eventually aims to reach the Q-value estimated by target network 420 by the end of the training stage. The weights of online network 406 are updated by optimizing the mean squared error loss as defined in Equation (1) and the weights of target network 420 are updated periodically with the weights of online network 406 after every N number of training episodes.

$$L_d = \mathbb{E}\left[\left\|\left(r_t + \gamma Q^{\pi_v}(S_{t+1}, \pi_v(S_{t+1}); \phi)\right) - Q(S_t, a_v; \theta)\right\|^2\right] \qquad (1)$$

where $(r_t + \gamma Q^{\pi v}(S_{t+1}, \pi_v(S_{t+1}); \phi))$ is the Q-value estimated by target network $\phi$ 420 and $Q(S_t, a_v; \theta)$ is the Q-value estimated by online network 406. $\gamma$ denotes the discount factor used in the cumulative reward estimation.

In one embodiment, HER (hindsight experience replay) may be used with the DDQN to provide performance gains for sparse reward problems. For HER, final states that do not reach the terminal state are considered to be an additional "terminal" state if they satisfy clinical constraints 1, 2, and 3 in Table 1 but do not satisfy clinical constraint 4 requiring distance $d_{OAR}$ between organs at risk and the ablation electrode is at least, e.g., 12 mm. Distance $d_{OAR}$ must be between 0 and 12mm in this embodiment.

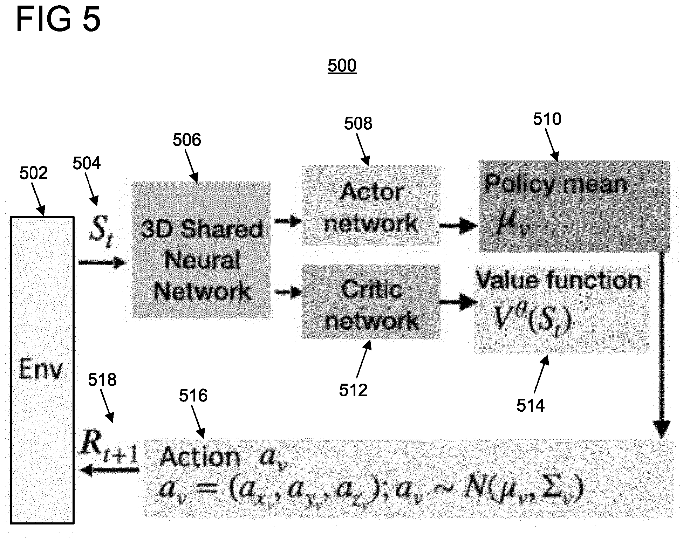

In one embodiment, the one or more actions $a_t$ comprise a continuous action modeled using policy gradient method with the AI agents implemented using PPO (proximal policy optimization). Figure 5 shows a framework 500 for determining a continuous action using PPO, in accordance with one or more embodiments. Framework 500 comprises a 3D shared neural network 506 and two smaller dense layer networks: actor network 508 and critic network 512. 3D shared neural network 506 receives current state $S_t$ 504 observed in environment 502 as input and extracts latent features from current state $S_t$ 504 as output. The latent features represent the most relevant or important features of current state $S_t$ 504 in a latent space of smaller dimensionality. Actor network 508 and critic network 512 receive the output of 3D shared neural network 506 as input and respectively generate policy mean $\mu_v$ 510 and value function $V^\theta(S_t)$ 514 as output. Policy mean $\mu_v$ is a multidimensional (with three values for the 3D coordinates of electrode skin endpoint $P_v$) representing a mean of the optimal action policy to be applied to reach an optimal state. Value function $V^\theta(S_t)$ 514 is a multivariate Gaussian distribution $N(\mu_v, \Sigma_v)$, where mean value $\mu_v$ is policy mean $\mu_v$ 510 output from actor network 508 and $\Sigma_v$ is a fixed variance value. Value function $V^\theta(S_t)$ 514 indicates how well the action is in relation to the considered action policy and how to adjust the action to improve the next action where the terminal state is not reached. A random continuous action value $a_v = (a_{xv}, a_{yv}, a_{zv})$ sampled from the Gaussian distribution $N(\mu_v, \Sigma_v)$ and applied to determine updated positions of electrode skin endpoint $P_{v+1} = P_v(a_v) = (x_v + a_{xv}, y_v + a_{yv}, z_v + a_{zv})$ in environment 502 and an updated net cumulative reward $r_{t+1}$ 518.

The net loss for training the 3D shared neural network 506, actor network 508, and critic network 512 is defined in Equation (2):

$$L_c = \mathbb{E}_t\left[L_{clip}(\theta) - c_1 L_t^{VF}(\theta) + c_2 S[\pi_v](S_t)\right] \qquad (2)$$

The first term of Equation (2) is a clipped loss $L_{clip}(\theta) = \mathbb{E}_t\left[\min(r_t(\theta), 1 - \varepsilon, 1 + \varepsilon)\hat{A}_t\right]$, where $\varepsilon$ controls the change in policy, $r_t$ is the ratio of likelihood of actions under current vs. old policy defined as $r_t(\theta) = \dfrac{\pi_\theta(a_t|s_t)}{\pi_{\theta_{old}}(a_t|s_t)}$, and $\hat{A}_t$ is an advantage function that measures the relative positive or negative reward value for the current set of actions with respect to an average set of actions. $\hat{A}_t$ is defined as $\hat{A}_t = \hat{R}_t - V^\theta(S_t)$, where $\hat{R}_t$ is the cumulative net rewards given by $\hat{R}_t = r_t + \gamma * r_{t+1} + \cdots + \gamma^{T-t} V^\theta(S_t)$ and $T$ is the maximum number of steps allowed in an episode.

The second term of Equation (2) is the mean squared error of value function $L_t^{VF}(\theta) = \left\|\hat{R}_t - V^\theta(S_t)\right\|^2$. The hyper-parameter $c_1$ controls the contribution of this loss term from critic network 512.

The third term of Equation (2) is the entropy term that dictates policy exploration with the hyper-parameter $c_2$,

where a lower $c_2$ value indicates lower exploration and vice versa.

At step 110 of Figure 1, a next state $S_{t+1}$ of the environment is defined based on the mask and the one or more updated positions of the respective ablation electrode. Next state $S_{t+1}$ is defined as $S_{t+1} = (m, P_u, P_{v+1})$ and the net cumulative reward $r_t$ is updated to $r_{t+1}$. In one example, as shown in Figure 2, next state $S_{t+1}$ 212 of environment 206 and an updated cumulative net reward $r_{t+1}$ are defined.

At step 112 of Figure 1, the steps of determining the one or more actions (step 108) and defining the next state (step 110) are repeated for a plurality of iterations to iteratively update the one or more current positions of the respective ablation electrode using 1) the next state as the current state and 2) the one or more updated positions as the one or more current positions to determine one or more final positions of the respective ablation electrode for performing an ablation on the one or more tumors. The ablation may be, for example, a radiofrequency ablation, a microwave ablation, a laser ablation, a cryoablation ablation, or an ablation performed using any other suitable technique.

Steps 108-110 are repeated until a stopping condition is reached. In one embodiment, the stopping condition is that the current state $S_t$ reaches a terminal or final state, which occurs when the value of the net cumulative reward $r_t$ reaches a predetermined threshold value indicating that all clinical constraints are satisfied. The clinical constraints in Table 1 are satisfied when the net cumulative reward $r_t$ is 2.12 with $r_d \geq 0.12$ and $r_l > 0$. In another embodiment, the stopping condition may be a predetermined number of iterations. In one example, as shown in Figure 2, workflow 200 is repeated using next state $S_{t+1}$ 212 as current state $S_t$ 208 and updated net cumulative reward $r_{t+1}$ 214 as current net cumulative reward $r_t$ 210.

At step 114 of Figure 1, the one or more final positions of each respective ablation electrode are output. For example, the one or more final positions of each respective ablation electrode can be output by displaying the one or more final positions on a display device of a computer system, storing the one or more final positions on a memory or storage of a computer system, or by transmitting the one or more final positions to a remote computer system. In one embodiment, the one or more final positions of each respective ablation electrode may be output to an end-to-end system for comprehensive planning, guidance, and verification of tumor thermal ablation.

Embodiments described with respect to method 100 of Figure 1 were experimentally validated using the LiTS (liver tumor segmentation) dataset comprising 130 CT volumes with expert annotations for tumors and livers. Each patient's CT volume comprises multiple tumors. A maximum of 10 tumors per patient were selected for ablation from predefined radium values with no collision of the tumor with any organ at risk. This resulted in a total of 496 cases, which were split into training, validation, and testing sets comprising 225, 131, and 140 cases respectively.

Pre-processing: First the segmentation of the organs at risk, blood vessels, and 9 segments of the liver are generated automatically using a deep learning image-to-image network. A combined 3D volume was defined with the tumor, liver, organs at risk, and skin masks. Each volume was constructed by applying the following steps sequentially. First, a dilation of 1 mm was applied to the ribs, skeleton, and blood vessels in the liver and a dilation of 5 mm was applied to organs at risk. Second, the ablation sphere radius for the tumor was computed at 1 mm resolution. Third, the masks were resampled to 3 mm. Fourth, the volumes were cropped to reduce its dimensions and remove unnecessary entry points using a liver mask in a perpendicular direction to an axial plane and from the back. Fifth, a distance map to the organs at risk were computed, excluding blood vessels in the liver. Finally, all volumes and distance maps were cropped to a dimension of (96,90,128).

Network architecture: The 3D network has the same architecture for both DDQN and PPO approaches. It has three 3D convolution layers with filter, kernel size, and strides of (32,8,4), (64,4,2), and (64,3,1) respectively. The resultant output was flattened and passed through a dense layer with 512 units output. All layers have ReLU (rectified linear unit) activations. For the DDΓaN, a dense layers network was used that receives as input the 512 units as input and returns 27 values corresponding to Q-values. For the PPO, two outputs result from the actor and critic networks following the shared network. The actor network has two dense layers with first a dense layer of 64 outputs, followed by ReLU, and lastly with a dense layer of 3 output values (mean values). Similarly, the critic network has two layers with a dense layer of 64 outputs, followed by ReLU, and finally a final dense layer of 1 output value (value estimate).

Training details: For the DDΓaN, in each episode, a random patient was sampled and the terminal state was attempted to be reached within a maximum of 50 steps by either exploration (randomly sampled action out of all possible actions) or exploitation (optimal action predicted by the online network). The experiences are populated in an experience replay buffer that stores all the (state, action, next state, reward) pairs in memory. At the start, exploration was performed more frequently and experiences were accumulated. After reaching a predefined number of experiences, in each episode, the online network is trained on a batch of randomly sampled experiences from the replay buffer with the loss given in Equation (1). Batch size was set to 32 and learning rate to 5e-4. Five values of $\gamma$ were evaluated: 0.1, 0.2, 0.3, 0.4, 0.5. The exploration and exploitation are controlled by a variable $\varepsilon$ initially set as 1, which decays with a decay rate of 0.9995. At the start of training, more exploration is performed while towards the end, more exploitation is performed. The target network weights $\phi$ are updated with the network weights of the online network $\theta$ periodically every 10 episodes. It was found that training the networks for 2000 episodes led to a stable convergence.

For the PPO, in each episode, a patient was randomly sampled. The electrode skin endpoint $P_v$ is displaced

to reach the terminal state within 50 steps. The network is updated at the end of the episode with the loss based on this episode s steps. In each episode, a joint optimization is performed with both the first and second loss terms of Equation (2), as performance gains were not observed using the third term of entropy loss with $c_2$ set to 0. The network was trained for 2000 episodes with a learning rate of 5e-4. The hyper-parameters $c_1$, $\Sigma_v$, and $\varepsilon$ (the PPO clip value) were empirically set to 1, .05, and 0.2 respectively. In an episode, the network updates were stopped when the mean KL (Kullback-Leibler) divergence estimate (the ratio of previous log probabilities to new log probabilities) for a given training example exceeds a threshold value set to 0.02.

Evaluation: For each test patient, 10 random initializations of the electrode skin endpoint $P_v$ were considered. The corresponding state $S_t$ is passed through the trained network, which either reaches a valid solution (terminal state, satisfying all clinical constraints) within 50 steps or not. If one or more valid solutions are found, the accuracy is set to 1, else to 0 (failure case). When multiple valid solutions are found, the final solution is chosen to be the one with the lowest electrode length. The model used for evaluation is the one that yields the highest accuracy of the validation set during all the training episodes.

In one embodiment, method 100 of Figure 1 may be performed using a plurality of AI agents to determine positions of a respective one of a plurality of ablation probes for performing thermal ablation on one or more tumors. In this embodiment, multi-agent reinforcement learning (MARL) was utilized for training the AI agents. In MARL, the AI agents collaborate to maximize the cumulative rewards by learning optimal policies used by the individual AI agents to select a set of actions to go from a current state to a terminal state. To foster collaboration between AI agents, a VDN (value decomposition networks) approach may be used in which DQN-style agents select and execute actions independent, but receive a joint reward computed on the overall state. The underlying assumption being that the joint action-value function can be decomposed as a sum of individual agent terms. The individual Q functions are consequently learned implicitly by backpropagation to maximize the joint action value function. Advantageously, the centralized training with the joint reward enforces collaboration between agents. Additionally, the execution is decentralized with each agent following a policy based on individual action value estimates, thus limiting the action space size as well as the reward complexity.

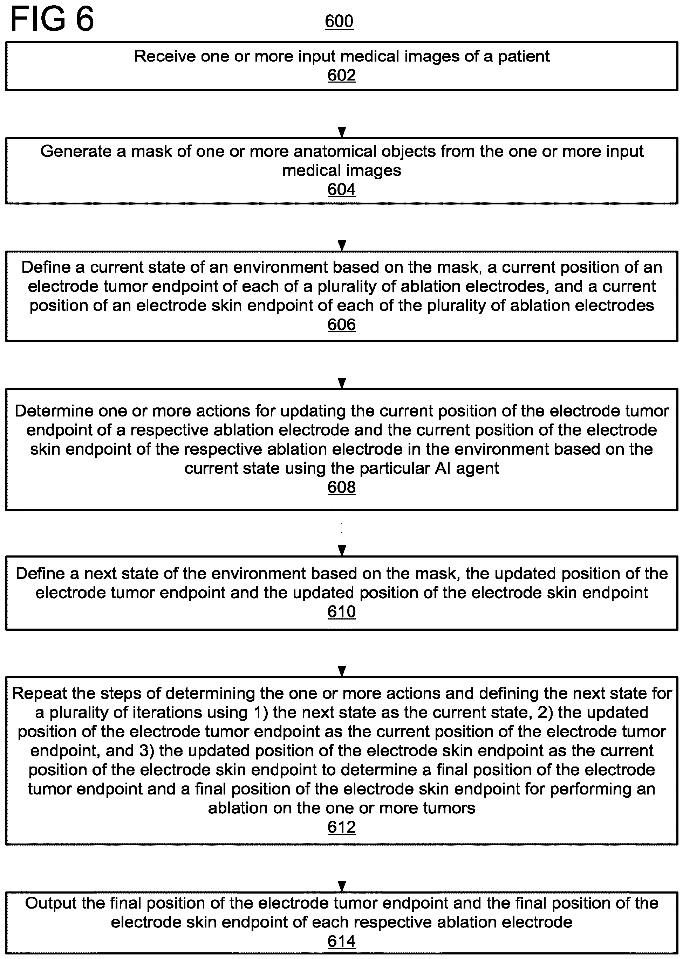

Figure 6 shows a method 600 for respectively determining a position of a plurality of ablation electrodes for performing a thermal ablation on one or more tumors, in accordance with one or more embodiments. The steps of method 600 of Figure 6 correspond to the steps of method 100 of Figure 1 using a plurality of AI agents. The steps of method 600 may be performed by one or more suitable computing devices, such as, e.g., computer 1102 of Figure 110.

At step 602 of Figure 6, one or more input medical images of a patient are received.

At step 604 of Figure 6, a mask of one or more anatomical objects is generated from the one or more input medical images. The one or more anatomical objects comprise one or more tumors.

At step 606 of Figure 6, a current state $S_t$ of an environment is defined based on the mask, a current position of an electrode tumor endpoint $P_u$ of each of a plurality of ablation electrodes, and a current position of an electrode skin endpoint $P_v$ of each of the plurality of ablation electrodes. In one embodiment, current state $S_t$ is further defined based on an ablation zone $A_i$ for ablation electrode $i$. The ablation zone $A_i$ may be modeled using any suitable shape. In one embodiment, ablation zone $A_i$ is modeled as an ellipsoid parameterized by the semi-axes a, b, and c, where b=c and a>b. Modeling ablation zone $A_i$ as an ellipsoid results in more complex problem solving as compared to spherical modeling since the ellipsoid removes many symmetries. As a result, the electrode tumor endpoint $P_u$ cannot be assumed to be fixed at the center of the tumor and must be optimized as well, thus increasing the action space size. The current state $S_t$ is therefore defined as $S_t = (m, P_{ui}, P_{vi}, A_i)$.

Steps 608-612 of Figure 6 are performed for each particular AI agent of the plurality of AI agents. Each particular AI agent iteratively updates one or more positions of a respective ablation electrode of the plurality of ablation electrodes for performing a thermal ablation on one or more tumors. Instead of each particular AI agent receiving a partial state of the environment, each of the plurality of AI agents receives the same current state $S_t$ of the environment.

At step 608 of Figure 6, one or more actions $a_t$ for updating the current position of the electrode tumor endpoint of a respective ablation electrode of the plurality of ablation electrodes and the current position of the electrode skin endpoint of the respective ablation electrode in the environment based on the current state using the particular AI agent. The one or more actions $a_t$ are determined based on a net cumulative reward $r_t$. The reward $r_t$ is defined based on clinical constraints.

The particular AI agent estimates action-value functions $Q_i(s, a_i)$ for each action. The action $a_t$ with the action-value that maximizes the net cumulative reward $r_t$ is selected. Each particular AI agent select the one or more actions $a_t$ individually based on a net cumulative reward $r_t$ for all AI agents. The one or more actions $a_t$ update or displace the current position of electrode tumor endpoint $P_{ui}$ of the respective ablation electrode $i$ and the current position of electrode skin endpoint $P_{vi}$ of the respective ablation electrode $i$ in the environment to an updated position of electrode tumor endpoint $P_{(u+1)i}$ and electrode skin endpoint $P_{(v+1)i}$ in the environment. In one embodiment, the one or more actions $a_t$ comprise a discrete set of predefined actions modeled using a value learning approach with a DDQN. In another embodiment, the one or more actions $a_t$ comprise a continuous action modeled using policy gradient method PPO.

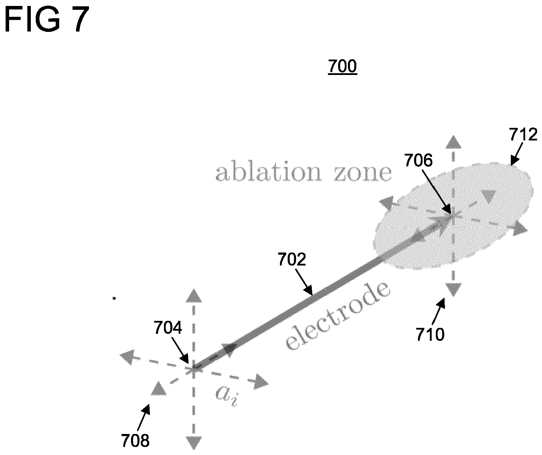

Figure 7 shows a visualization of an action space 700 of an ablation electrode, in accordance with one or more embodiments. In action space 700, denoted $A = \{a_i\}$ where $1 \leq i \leq 24$, ablation electrode 702 comprises an electrode tumor endpoint $P_u$ 706 and electrode skin endpoint $P_v$ 704. Electrode tumor endpoint $P_u$ 706 and electrode skin endpoint $P_v$ 704 may be respectively displaced by, e.g., 1 or 5 voxels in any direction of a fixed Cartesian coordinate system 710 and 708. Ablation zone 712 is modeled as an ellipsoid with one long axis and two smaller axes of equal size.

At step 610 of Figure 6, a next state $S_{t+1}$ of the environment is defined based on the mask, the updated position of the electrode tumor endpoint of the respective ablation electrode, and the updated position of the electrode skin endpoint of the respective ablation electrode. Next state $S_{t+1}$ is defined as $S_{t+1} = (m, P_{(u+1)i}, P_{(v+1)i})$.

At step 612 of Figure 6, the steps of determining the one or more actions (step 608) and defining the next state (step 610) are repeated for a plurality of iterations using 1) the next state as the current state, 2) the updated position of the electrode tumor endpoint as the current position of the electrode tumor endpoint, and 3) the updated position of the electrode skin endpoint as the current position of the electrode skin endpoint to determine a final position of the electrode tumor endpoint and a final position of the electrode skin endpoint for performing an ablation on the one or more tumors. Steps 608-610 are repeated until a stopping condition is reached.

At step 614 of Figure 6, the final position of the electrode tumor endpoint and the final position of the electrode skin endpoint of each respective ablation electrode are output.

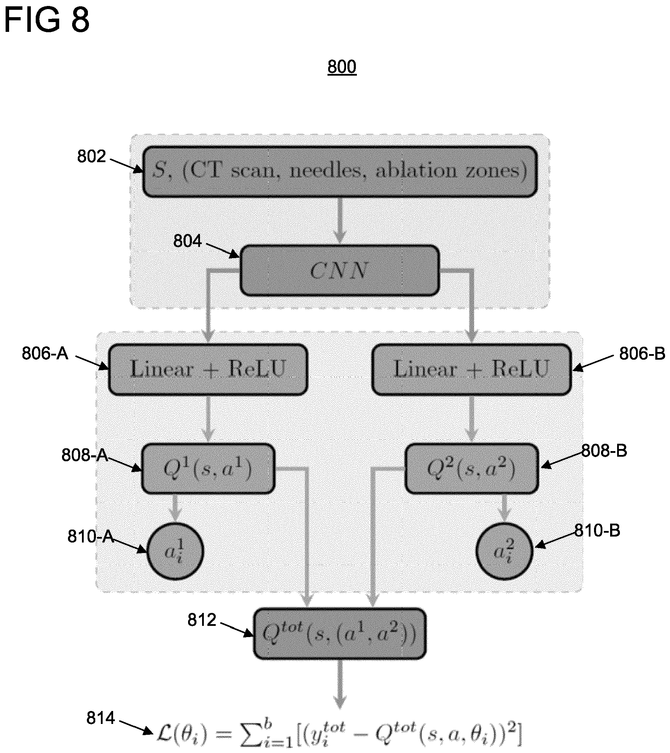

Figure 8 shows a workflow 800 of a MARL implementation for determining an optimal position of two ablation electrodes for performing a thermal ablation on one or more tumors, in accordance with one or more embodiments.

A current state 802, defined by the CT scan, one or more positions of the ablation electrodes, and the ablation zones, is received as input by CNN (convolutional neural network) 804. CNN 804 extracts information from the CT scan that is relevant for both ablation electrodes. The output of CNN 804 is respectively received as input by linear+ReLU layers 806-A and 806-B. Each linear+ReLU layer 806-A and 806-B extracts information that is relevant for its corresponding ablation electrode to generate Q-values $Q^1(s, a^1)$ 808-A and $Q^2(s, a^2)$ 808-B for selecting actions $a_i^1$ 810-A and $a_i^2$ 810-B. Q-values $Q^1(s, a^1)$ 808-A and $Q^2(s, a^2)$ 808-B are combined to $Q^{tot}(s, (a^1, a^2))$ 812. The AI agent is trained during an offline training stage by gradient descent using the loss function 814 defined in Equation (3) computed on a joint action-value function $Q^{tot}$:

$$L(\theta_i) = \sum_{i=1}^{b} \left[ \left( y_i^{tot} - Q^{tot}(s, a, \theta_i) \right)^2 \right] \qquad (2)$$

Training with training data having different tumor shapes, sizes, and locations should give the best results. To overcome the limitation in the number of available training data, the training data may be extended by generating synthetic data. Once trained, the trained AI agents may be applied during an inference stage (e.g., to perform method 600 of Figure 6).

Embodiments described herein allow for the standardizing of procedures as thermal ablation planning and guidance will become repeatable, operator-independent, less complex, and less time consuming.

In one embodiment, ablation parameters for performing the tumor thermal ablation are also determined. A plurality of AI agents is trained for different ablation scenarios according to different ablation parameters (e.g., different ablation power and different ablation duration resulting in different ablation zones, which may be represented as a sphere or ellipsoid). During the inference stage, each of the plurality of AI agents are executed in parallel. For example, a first set of the plurality of AI agents may be executed to determine an electrode tumor endpoint and/or a second set of the plurality of AI agents may be executed to determine an electrode skin endpoint (e.g., to perform method 100 of Figure 1 and method 600 of Figure 6) and the AI agent(s) (e.g., from the first set and/or from the second set) associated with the Pareto optimal solution may be selected to determine optimal ablation parameters.

In one embodiment, intraoperative guidance for performing a thermal ablation on the one or more tumors are generated based on the final positions of the ablation electrodes (e.g., determined according to method 100 of Figure 1 or method 600 of Figure 6). For example, the final positions of the ablation electrodes may be output to an end-to-end system for comprehensive planning, guidance, and verification of tumor thermal ablation. After the acquisition of patient CT images, various anatomical structures (such as, e.g., organs at risk (e.g., ribs, kidneys, etc.), important blood vessels, and tumors) may be segmented. Based on the final positions of the ablation electrodes and the segmented anatomical structures, the system may provide various guidance and planning options. This automatic guidance and planning feature can be part of an end-to-end integrated system for needle-based procedures, making planning information available during the treatment selection process and the procedure.

In one embodiment, the guidance and validation may be implemented using augmented reality combined with electromagnetic (EM) tracker technologies. In another embodiment, the guidance and validation may be implemented using a laser pointer pointing at, e.g., the one or more final positions and the direction (e.g., determined according to method 100 of Figure 1 or method 600 of Figure 6), therefore showing the angle of insertion in the case of CT guidance or ultrasound guidance. In another embodiment, guidance and validation may be implemented using ultrasound or CT guidance, where the ultrasound or CT image is combined with (e.g., co-registered with) one or more preoperative planning images. The co-registration would allow the presentation of the same preoperative information used to generate the operative plan during the procedure. For example, a planned trajectory may be overlaid on the real time ultrasound or CT images. The registration accuracy can further be improved by taking into account the breathing motion of the patient. A surrogate signal obtained using a 3D camera that determines the breathing phase may be used. The guidance would therefore show to a clinical user the entry point on the skin of a patient, the direction and angle to be used for inserting the ablation needle, the final target point within the tumor, as well as the final targeted ablation zone.

Embodiments described herein allow the real-time visualization of the plan during the intervention to facilitate the realization of the planned probe trajectory. The system may display the pre-operative CT images, together with the organ segmentation masks (e.g., liver, hepatic vessels, tumor, organs at risk), enabling control of the organs that the electrode is travelling through. The optimal entry and target points defined during the planning phase may also be rendered.

In one embodiment, by bringing the planning information into the intra-operative space, the ablation verification may be performed at the end of the procedure. Once the ablation is performed, a post-operative image with contrast media in the parenchyma may be acquired to observe the ablated area and verify its extent. The ablation zone is automatically segmented on the post-ablation control CT image, which is registered to the pre-operative CT images, allowing for the estimation of the ablation margin immediately and accurately. The system fuses this post-operative image to a pre-operative image acquired before ablation where the tumor is visible to assess the success of the procedure. The minimal distance between the ablation border and the tumor border is measured, and is should be greater than 5 mm for the ablation to be considered a success. As this is performed during the procedure, it allows for the immediate correction of the margin by planning a consecutive ablation to achieve a complete ablation. This reduces the need for repeated sessions.

Embodiments described herein are described with respect to the claimed systems as well as with respect to the claimed methods. Features, advantages or alternative embodiments herein can be assigned to the other claimed objects and vice versa. In other words, claims for the systems can be improved with features described or claimed in the context of the methods. In this case, the functional features of the method are embodied by objective units of the providing system.

Furthermore, certain embodiments described herein are described with respect to methods and systems utilizing trained machine learning based networks (or models), as well as with respect to methods and systems for training machine learning based networks. Features, advantages or alternative embodiments herein can be assigned to the other claimed objects and vice versa. In other words, claims for methods and systems for training a machine learning based network can be improved with features described or claimed in context of the methods and systems for utilizing a trained machine learning based network, and vice versa.

In particular, the trained machine learning based networks applied in embodiments described herein can be adapted by the methods and systems for training the machine learning based networks. Furthermore, the input data of the trained machine learning based network can comprise advantageous features and embodiments of the training input data, and vice versa. Furthermore, the output data of the trained machine learning based network can comprise advantageous features and embodiments of the output training data, and vice versa.

In general, a trained machine learning based network mimics cognitive functions that humans associate with other human minds. In particular, by training based on training data, the trained machine learning based network is able to adapt to new circumstances and to detect and extrapolate patterns.

In general, parameters of a machine learning based network can be adapted by means of training. In particular, supervised training, semi-supervised training, unsupervised training, reinforcement learning and/or active learning can be used. Furthermore, representation learning (an alternative term is "feature learning") can be used. In particular, the parameters of the trained machine learning based network can be adapted iteratively by several steps of training.

In particular, a trained machine learning based network can comprise a neural network, a support vector machine, a decision tree, and/or a Bayesian network, and/or the trained machine learning based network can be based on k-means clustering, Q-learning, genetic algorithms, and/or association rules. In particular, a neural network can be a deep neural network, a convolutional neural network, or a convolutional deep neural network. Furthermore, a neural network can be an adversarial network, a deep adversarial network and/or a generative adversarial network.

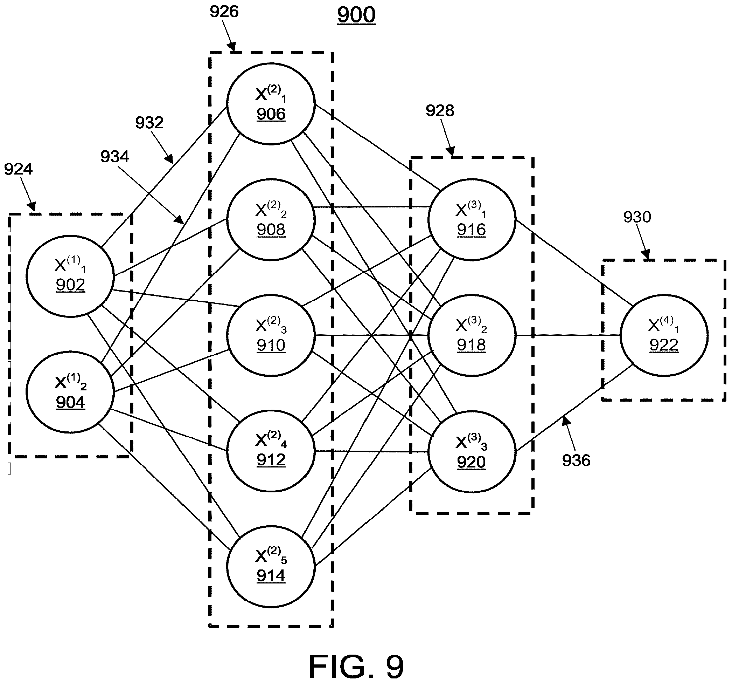

Figure 9 shows an embodiment of an artificial neural network 900, in accordance with one or more embodiments. Alternative terms for "artificial neural network" are "neural network", "artificial neural net" or "neural net". Machine learning networks described herein, such as, e.g., the AI agents, may be implemented using artificial neural network 900.

The artificial neural network 900 comprises nodes 902-922 and edges 932, 934, ..., 936, wherein each edge 932, 934, ..., 936 is a directed connection from a first node 902-922 to a second node 902-922. In general, the first node 902-922 and the second node 902-922 are different nodes 902-922, it is also possible that the first node 902-922 and the second node 902-922 are identical. For example, in Figure 9, the edge 932 is a directed connection from the node 902 to the node 906, and the edge 934 is a directed connection from the node 904 to the node 906. An edge 932, 934, ..., 936 from a first node 902-922 to a second node 902-922 is also denoted as "ingoing edge" for the second node 902-922 and as "outgoing edge" for the first node 902-922.

In this embodiment, the nodes 902-922 of the artificial neural network 900 can be arranged in layers 924-930, wherein the layers can comprise an intrinsic order introduced by the edges 932, 934, ..., 936 between the nodes 902-922. In particular, edges 932, 934, ..., 936 can exist only between neighboring layers of nodes. In the embodiment shown in Figure 9, there is an input layer 924 comprising only nodes 902 and 904 without an incoming edge, an output layer 930 comprising only node 922 without outgoing edges, and hidden layers 926, 928 in-between the input layer 924 and the output layer 930. In general, the number of hidden layers 926, 928 can be chosen arbitrarily. The number of nodes 902 and 904 within the input layer 924 usually relates to the number of input values of the neural network 900, and the number of nodes 922 within the output layer 930 usually relates to the number of output values of the neural network 900.

In particular, a (real) number can be assigned as a value to every node 902-922 of the neural network 900. Here, $x^{(n)}_i$ denotes the value of the i-th node 902-922 of the n-th layer 924-930. The values of the nodes 902-922 of the input layer 924 are equivalent to the input values of the neural network 900, the value of the node 922 of the output layer 930 is equivalent to the output value of the neural network 900. Furthermore, each edge 932, 934, ..., 936 can comprise a weight being a real number, in particular, the weight is a real number within the interval [-1, 1] or within the interval [0, 1]. Here, $w^{(m,n)}_{i,j}$ denotes the weight of the edge between the i-th node 902-922 of the m-th layer 924-930 and the j-th node 902-922 of the n-th layer 924-930. Furthermore, the abbreviation $w^{(n)}_{i,j}$ is defined for the weight $w^{(n,n+1)}_{i,j}$.

In particular, to calculate the output values of the neural network 900, the input values are propagated through the neural network. In particular, the values of the nodes 902-922 of the (n+1)-th layer 924-930 can be calculated based on the values of the nodes 902-922 of the n-th layer 924-930 by

$$x^{(n+1)}_j = f\left(\sum_i x^{(n)}_i \cdot w^{(n)}_{i,j}\right).$$

Herein, the function f is a transfer function (another term is "activation function"). Known transfer functions are step functions, sigmoid function (e.g. the logistic function, the generalized logistic function, the hyperbolic tangent, the Arctangent function, the error function, the smoothstep function) or rectifier functions. The transfer function is mainly used for normalization purposes.

In particular, the values are propagated layer-wise through the neural network, wherein values of the input layer 924 are given by the input of the neural network 900, wherein values of the first hidden layer 926 can be calculated based on the values of the input layer 924 of the neural network, wherein values of the second hidden layer 928 can be calculated based in the values of the first hidden layer 926, etc.

In order to set the values $w^{(m,n)}_{i,j}$ for the edges, the neural network 900 has to be trained using training data. In particular, training data comprises training input data and training output data (denoted as $t_i$). For a training step, the neural network 900 is applied to the training input data to generate calculated output data. In particular, the training data and the calculated output data comprise a number of values, said number being equal with the number of nodes of the output layer.

In particular, a comparison between the calculated output data and the training data is used to recursively adapt the weights within the neural network 900 (backpropagation algorithm). In particular, the weights are changed according to

$$w'^{(n)}_{i,j} = w^{(n)}_{i,j} - \gamma \cdot \delta^{(n)}_j \cdot x^{(n)}_i$$

wherein $\gamma$ is a learning rate, and the numbers $\delta^{(n)}_j$ can be recursively calculated as

$$\delta^{(n)}_j = \left(\sum_k \delta^{(n+1)}_k \cdot w^{(n+1)}_{j,k}\right) \cdot f'\left(\sum_i x^{(n)}_i \cdot w^{(n)}_{i,j}\right)$$

based on $\delta^{(n+1)}_j$, if the (n+1)-th layer is not the output layer, and

$$\delta_j^{(n)} = \left( x_k^{(n+1)} \cdot t_j^{(n+1)} \right) \cdot f' \left( \sum_i x_i^{(n)} \cdot w_{i,j}^{(n)} \right)$$

if the (n+1)-th layer is the output layer 930, wherein f is the first derivative of the activation function, and y(n+1)j is the comparison training value for the j-th node of the output layer 930.

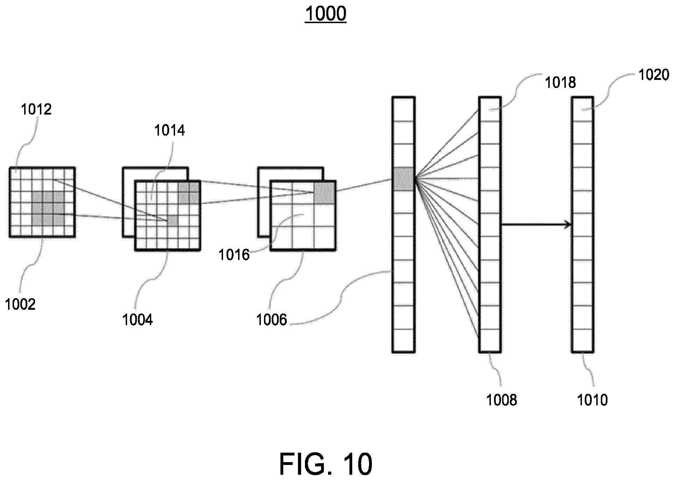

Figure 10 shows a convolutional neural network 1000, in accordance with one or more embodiments. Machine learning networks described herein, such as, e.g., the AI agents, may be implemented using convolutional neural network 1000.

In the embodiment shown in Figure 10, the convolutional neural network comprises 1000 an input layer 1002, a convolutional layer 1004, a pooling layer 1006, a fully connected layer 1008, and an output layer 1010. Alternatively, the convolutional neural network 1000 can comprise several convolutional layers 1004, several pooling layers 1006, and several fully connected layers 1008, as well as other types of layers. The order of the layers can be chosen arbitrarily, usually fully connected layers 1008 are used as the last layers before the output layer 1010.

In particular, within a convolutional neural network 1000, the nodes 1012-1020 of one layer 1002-1010 can be considered to be arranged as a d-dimensional matrix or as a d-dimensional image. In particular, in the two-dimensional case the value of the node 1012-1020 indexed with i and j in the n-th layer 1002-1010 can be denoted as x(n)[i,j]. However, the arrangement of the nodes 1012-1020 of one layer 1002-1010 does not have an effect on the calculations executed within the convolutional neural network 1000 as such, since these are given solely by the structure and the weights of the edges.

In particular, a convolutional layer 1004 is characterized by the structure and the weights of the incoming edges forming a convolution operation based on a certain number of kernels. In particular, the structure and the weights of the incoming edges are chosen such that the values x(n)k of the nodes 1014 of the convolutional layer 1004 are calculated as a convolution x(n)k = Kk * x(n-1) based on the values x(n-1) of the nodes 1012 of the preceding layer 1002, where the convolution * is defined in the two-dimensional case as

$$x_k^{(n)}[i,j] = \left( K_k * x^{(n-1)} \right)[i,j] = \sum_{i'} \sum_{j'} K_k[i',j'] \cdot x^{(n-1)}[i-i', j-j'].$$

Here the k-th kernel Kk is a d-dimensional matrix (in this embodiment a two-dimensional matrix), which is usually small compared to the number of nodes 1012-1018 (e.g. a 3x3 matrix, or a 5x5 matrix). In particular, this implies that the weights of the incoming edges are not independent, but chosen such that they produce said convolution equation. In particular, for a kernel being a 3x3 matrix, there are only 9 independent weights (each entry of the kernel matrix corresponding to one independent weight), irrespectively of the number of nodes 1012-1020 in the respective layer 1002-1010. In particular, for a convolutional layer 1004, the number of nodes 1014 in the convolutional layer is equivalent to the number of nodes 1012 in the preceding layer 1002 multiplied with the number of kernels.

If the nodes 1012 of the preceding layer 1002 are arranged as a d-dimensional matrix, using a plurality of kernels can be interpreted as adding a further dimension (denoted as "depth" dimension), so that the nodes 1014 of the convolutional layer 1004 are arranged as a (d+1)-dimensional matrix. If the nodes 1012 of the preceding layer 1002 are already arranged as a (d+1)-dimensional matrix comprising a depth dimension, using a plurality of kernels can be interpreted as expanding along the depth dimension, so that the nodes 1014 of the convolutional layer 1004 are arranged also as a (d+1)-dimensional matrix, wherein the size of the (d+1)-dimensional matrix with respect to the depth dimension is by a factor of the number of kernels larger than in the preceding layer 1002.

The advantage of using convolutional layers 1004 is that spatially local correlation of the input data can exploited by enforcing a local connectivity pattern between nodes of adjacent layers, in particular by each node being connected to only a small region of the nodes of the preceding layer.

In embodiment shown in Figure 10, the input layer 1002 comprises 36 nodes 1012, arranged as a two-dimensional 6x6 matrix. The convolutional layer 1004 comprises 72 nodes 1014, arranged as two two-dimensional 6x6 matrices, each of the two matrices being the result of a convolution of the values of the input layer with a kernel. Equivalently, the nodes 1014 of the convolutional layer 1004 can be interpreted as arranges as a three-dimensional 6x6x2 matrix, wherein the last dimension is the depth dimension.

A pooling layer 1006 can be characterized by the structure and the weights of the incoming edges and the activation function of its nodes 1016 forming a pooling operation based on a non-linear pooling function f. For example, in the two dimensional case the values x(n) of the nodes 1016 of the pooling layer 1006 can be calculated based on the values x(n-1) of the nodes 1014 of the preceding layer 1004 as

$$x^{(n)}[i,j] = f(x^{(n-1)}[id_1, jd_2], ..., x^{(n-1)}[id_1+d_1-1, jd_2+d_2-1])$$

In other words, by using a pooling layer 1006, the number of nodes 1014, 1016 can be reduced, by replacing a number d1 d2 of neighboring nodes 1014 in the preceding layer 1004 with a single node 1016 being calculated as a function of the values of said number of neighboring nodes in the pooling layer. In particular, the pooling function f can be the max-function, the average or the L2-Norm. In particular, for a pooling layer 1006 the weights of the incoming edges are fixed and are not modified by training.

The advantage of using a pooling layer 1006 is that the number of nodes 1014, 1016 and the number of parameters is reduced. This leads to the amount of computation in the network being reduced and to a control of overfitting.

In the embodiment shown in Figure 10, the pooling layer 1006 is a max-pooling, replacing four neighboring nodes with only one node, the value being the maximum of the values of the four neighboring nodes. The max-pooling is applied to each d-dimensional matrix of the previous layer; in this embodiment, the max-pooling is applied to each of the two two-dimensional matrices, reducing the number of nodes from 72 to 18.

A fully-connected layer 1008 can be characterized by the fact that a majority, in particular, all edges between nodes 1016 of the previous layer 1006 and the nodes 1018 of the fully-connected layer 1008 are present, and wherein the weight of each of the edges can be adjusted individually.

In this embodiment, the nodes 1016 of the preceding layer 1006 of the fully-connected layer 1008 are displayed both as two-dimensional matrices, and additionally as non-related nodes (indicated as a line of nodes, wherein the number of nodes was reduced for a better presentability). In this embodiment, the number of nodes 1018 in the fully connected layer 1008 is equal to the number of nodes 1016 in the preceding layer 1006. Alternatively, the number of nodes 1016, 1018 can differ.

Furthermore, in this embodiment, the values of the nodes 1020 of the output layer 1010 are determined by applying the Softmax function onto the values of the nodes 1018 of the preceding layer 1008. By applying the Softmax function, the sum the values of all nodes 1020 of the output layer 1010 is 1, and all values of all nodes 1020 of the output layer are real numbers between 0 and 1.

A convolutional neural network 1000 can also comprise a ReLU (rectified linear units) layer or activation layers with non-linear transfer functions. In particular, the number of nodes and the structure of the nodes contained in a ReLU layer is equivalent to the number of nodes and the structure of the nodes contained in the preceding layer. In particular, the value of each node in the ReLU layer is calculated by applying a rectifying function to the value of the corresponding node of the preceding layer.

The input and output of different convolutional neural network blocks can be wired using summation (residual / dense neural networks), element-wise multiplication (attention) or other differentiable operators. Therefore, the convolutional neural network architecture can be nested rather than being sequential if the whole pipeline is differentiable.

In particular, convolutional neural networks 1000 can be trained based on the backpropagation algorithm. For preventing overfitting, methods of regularization can be used, e.g. dropout of nodes 1012-1020, stochastic pooling, use of artificial data, weight decay based on the L1 or the L2 norm, or max norm constraints. Different loss functions can be combined for training the same neural network to reflect the joint training objectives. A subset of the neural network parameters can be excluded from optimization to retain the weights pretrained on another datasets.

Systems, apparatuses, and methods described herein may be implemented using digital circuitry, or using one or more computers using well-known computer processors, memory units, storage devices, computer software, and other components. Typically, a computer includes a processor for executing instructions and one or more memories for storing instructions and data. A computer may also include, or be coupled to, one or more mass storage devices, such as one or more magnetic disks, internal hard disks and removable disks, magneto-optical disks, optical disks, etc.

Systems, apparatus, and methods described herein may be implemented using computers operating in a client-server relationship. Typically, in such a system, the client computers are located remotely from the server computer and interact via a network. The client-server relationship may be defined and controlled by computer programs running on the respective client and server computers.

Systems, apparatus, and methods described herein may be implemented within a network-based cloud computing system. In such a network-based cloud computing system, a server or another processor that is connected to a network communicates with one or more client computers via a network. A client computer may communicate with the server via a network browser application residing and operating on the client computer, for example. A client computer may store data on the server and access the data via the network. A client computer may transmit requests for data, or requests for online services, to the server via the network. The server may perform requested services and provide data to the client computer(s). The server may also transmit data adapted to cause a client computer to perform a specified function, e.g., to perform a calculation, to display specified data on a screen, etc. For example, the server may transmit a request adapted to cause a client computer to perform one or more of the steps or functions of the methods and workflows described herein, including one or more of the steps or functions of Figures 1 or 6. Certain steps or functions of the methods and workflows described herein, including one or more of the steps or functions of Figures 1 or 6, may be performed by a server or by another processor in a network-based cloud-computing system. Certain steps or functions of the methods and workflows described herein, including one or more of the steps of Figures 1 or 6, may be performed by a client computer in a network-based cloud computing system. The steps or functions of the methods and workflows described herein, including one or more of the steps of Figures 1 or 6, may be performed by a server and/or by a client computer in a network-based cloud computing system, in any combination.

Systems, apparatus, and methods described herein may be implemented using a computer program product tangibly embodied in an information carrier, e.g., in a non-transitory machine-readable storage device, for execution by a programmable processor; and the method and workflow steps described herein, including one or more of the steps or functions of Figures 1 or 6, may be implemented using one or more computer programs that are executable by such a processor. A computer program is a set of computer program instructions that can be used, directly or indirectly, in a computer to perform a certain activity or bring about a certain result. A computer program can be written in any form of programming language, including compiled or interpreted languages, and it can be deployed in any form, including as a stand-alone program or as a module, component, subroutine, or other unit suitable for use in a computing environment.

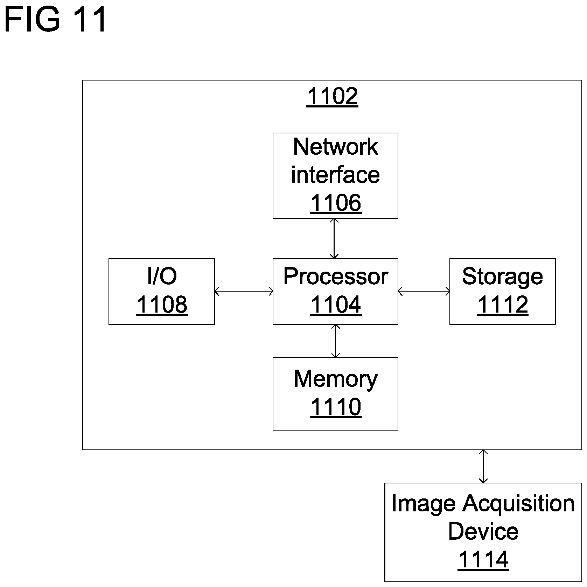

A high-level block diagram of an example computer 1102 that may be used to implement systems, apparatus, and methods described herein is depicted in Figure 11. Computer 1102 includes a processor 1104 operatively coupled to a data storage device 1112 and a memory 1110. Processor 1104 controls the overall operation of computer 1102 by executing computer program instructions that define such operations. The computer program instructions may be stored in data storage device 1112, or other computer readable medium, and loaded into memory 1110 when execution of the computer program instructions is desired. Thus, the method and workflow steps or functions of Figures 1 or 6 can be defined by the computer program instructions stored in memory 1110 and/or data storage device 1112 and controlled by processor 1104 executing the computer program instructions. For example, the computer program instructions can be implemented as computer executable code programmed by one skilled in the art to perform the method and workflow steps or functions of Figures 1 or 6. Accordingly, by executing the computer program instructions, the processor 1104 executes the method and workflow steps or functions of Figures 1 or 6. Computer 1102 may also include one or more network interfaces 1106 for communicating with other devices via a network. Computer 1102 may also include one or more input/output devices 1108 that enable user interaction with computer 1102 (e.g., display, keyboard, mouse, speakers, buttons, etc.).

Processor 1104 may include both general and special purpose microprocessors, and may be the sole processor or one of multiple processors of computer 1102. Processor 1104 may include one or more central processing units (CPUs), for example. Processor 1104, data storage device 1112, and/or memory 1110 may include, be supplemented by, or incorporated in, one or more application-specific integrated circuits (ASICs) and/or one or more field programmable gate arrays (FPGAs).

Data storage device 1112 and memory 1110 each include a tangible non-transitory computer readable storage medium. Data storage device 1112, and memory 1110, may each include high-speed random access memory, such as dynamic random access memory (DRAM), static random access memory (SRAM), double data rate synchronous dynamic random access memory (DDR RAM), or other random access solid state memory devices, and may include non-volatile memory, such as one or more magnetic disk storage devices such as internal hard disks and removable disks, magneto-optical disk storage devices, optical disk storage devices, flash memory devices, semiconductor memory devices, such as erasable programmable read-only memory (EPROM), electrically erasable programmable read-only memory (EEPROM), compact disc read-only memory (CD-ROM), digital versatile disc read-only memory (DVD-ROM) disks, or other non-volatile solid state storage devices.

Input/output devices 1108 may include peripherals, such as a printer, scanner, display screen, etc. For example, input/output devices 1108 may include a display device such as a cathode ray tube (CRT) or liquid crystal display (LCD) monitor for displaying information to the user, a keyboard, and a pointing device such as a mouse or a trackball by which the user can provide input to computer 1102.

An image acquisition device 1114 can be connected to the computer 1102 to input image data (e.g., medical images) to the computer 1102. It is possible to implement the image acquisition device 1114 and the computer 1102 as one device. It is also possible that the image acquisition device 1114 and the computer 1102 communicate wirelessly through a network. In a possible embodiment, the computer 1102 can be located remotely with respect to the image acquisition device 1114.

Any or all of the systems and apparatus discussed herein may be implemented using one or more computers such as computer 1102.

One skilled in the art will recognize that an implementation of an actual computer or computer system may have other structures and may contain other components as well, and that Figure 11 is a high level representation of some of the components of such a computer for illustrative purposes.

The foregoing Detailed Description is to be understood as being in every respect illustrative and exemplary, but not restrictive, and the scope of the invention disclosed herein is not to be determined from the Detailed Description, but rather from the claims as interpreted according to the full breadth permitted by the patent laws. It is to be understood that the embodiments shown and described herein are only illustrative of the principles of the present invention and that various modifications may be implemented by those skilled in the art without departing from the scope of the invention. Those skilled in the art could implement various other feature combinations without departing from the scope of the invention.

**Claims**

1. A computer-implemented method comprising:

   defining (606) a current state (802) of an environment based on a mask of one or more anatomical objects, a current position of an electrode tumor endpoint (706) of each of a plurality of ablation electrodes (702), and a current position of an electrode skin endpoint (704) of each of the plurality of ablation electrodes, the one or more anatomical objects comprising one or more tumors;
   for each particular AI (artificial intelligence) agent of a plurality of AI agents:

   determining (608) one or more actions (810) for updating the current position of the electrode tumor endpoint of a respective ablation electrode of the plurality of ablation electrodes and the current position of the electrode skin endpoint of the respective ablation electrode in the environment based on the current state using the particular AI agent,
   defining (610) a next state of the environment based on the mask, the updated position of the electrode tumor endpoint of the respective ablation electrode, and the updated position of the electrode skin endpoint of the respective ablation electrode, and
   repeating (612) the steps of determining the one or more actions and defining the next state for a plurality of iterations to iteratively update the current position of the electrode tumor endpoint and the current position of the electrode skin endpoint using 1) the next state as the current state, 2) the updated position of the electrode tumor endpoint of the respective ablation electrode as the current position of the electrode tumor endpoint of the respective ablation electrode, and 3) the updated position of the electrode skin endpoint of the respective ablation electrode as the current position of the electrode skin endpoint of the respective ablation electrode to determine a final position of the electrode tumor endpoint of the respective ablation electrode and a final position of the electrode skin endpoint of the respective ablation electrode for performing a thermal ablation on the one or more tumors; and
   outputting (614) the final position of the electrode tumor endpoint and the final position of the electrode skin endpoint of each of the plurality of ablation electrodes.

2. The computer-implemented method of claim 1, wherein:

   defining a next state of the environment comprises updating a joint net cumulative reward for the plurality of AI agents, wherein the joint net cumulative reward is defined based on clinical constraints; and
   repeating the steps of determining the one or more actions and defining the next state for a plurality of iterations comprises repeating the steps of determining the one or more actions and defining the next state until the joint net cumulative reward satisfies a threshold value.

3. The computer-implemented method of claim 1 or 2, wherein determining one or more actions for updating the current position of the electrode tumor endpoint of a respective ablation electrode of the plurality of ablation electrodes and the current position of the electrode skin endpoint of the respective ablation electrode in the environment based on the current state using the particular AI agent comprises:
   determining one or more discrete predefined actions using the particular AI agent implemented using a double deep Q network.

4. The computer-implemented method of any one of claims 1-3, wherein determining one or more actions for updating the current position of the electrode tumor endpoint of a respective ablation electrode of the plurality of ablation electrodes and the current position of the electrode skin endpoint of the respective ablation electrode in the environment based on the current state using the particular AI agent comprises:
   determining a continuous action using the particular AI agent implemented using proximal policy optimization.

5. The computer-implemented method of any one of claims 1-4, wherein defining a current state of an environment based on a mask of one or more anatomical objects, a current position of an electrode tumor endpoint of each of a plurality of ablation electrodes, and a current position of an electrode skin endpoint of each of the plurality of ablation electrodes comprises:
defining the current state of the environment based on an ablation zone (712) of each of the plurality of ablation electrodes, wherein the ablation zones are modeled as an ellipsoid.

6. The computer-implemented method of any one of claims 1-5, wherein determining one or more actions for updating the current position of the electrode tumor endpoint of a respective ablation electrode of the plurality of ablation electrodes and the current position of the electrode skin endpoint of the respective ablation electrode in the environment based on the current state using the particular AI agent comprises:
determining the one or more actions based on the same current state for the plurality of AI agents.

7. The computer-implemented method of claim 6, wherein determining one or more actions for updating the current position of the electrode tumor endpoint of a respective ablation electrode of the plurality of ablation electrodes and the current position of the electrode skin endpoint of the respective ablation electrode in the environment based on the current state using the particular AI agent comprises:
determining the one or more actions based on a joint net cumulative reward for the plurality of AI agents.

8. The computer-implemented method of any one of claims 1-7, further comprising:
generating intraoperative guidance for performing a thermal ablation on the one or more tumors based on the one or more final positions.

9. The computer-implemented method of any one of claims 1-8, wherein the plurality of AI agents is trained according to different ablation parameters, the method further comprising:
determining optimal ablation parameters for performing an ablation on the one or more tumors by selecting at least one of the plurality of AI agents.

10. An apparatus comprising:

means for defining (606) a current state (802) of an environment based on a mask of one or more anatomical objects, a current position of an electrode tumor endpoint (706) of each of a plurality of ablation electrodes (702), and a current position of an electrode skin endpoint (704) of each of the plurality of ablation electrodes, the one or more anatomical objects comprising one or more tumors;
for each particular AI (artificial intelligence) agent of a plurality of AI agents:

means for determining (608) one or more actions (810) for updating the current position of the electrode tumor endpoint of a respective ablation electrode of the plurality of ablation electrodes and the current position of the electrode skin endpoint of the respective ablation electrode in the environment based on the current state using the particular AI agent,
means for defining (610) a next state of the environment based on the mask, the updated position of the electrode tumor endpoint of the respective ablation electrode, and the updated position of the electrode skin endpoint of the respective ablation electrode, and
means for repeating (612) the steps of determining the one or more actions and defining the next state for a plurality of iterations to iteratively update the current position of the electrode tumor endpoint and the current position of the electrode skin endpoint using 1) the next state as the current state, 2) the updated position of the electrode tumor endpoint of the respective ablation electrode as the current position of the electrode tumor endpoint of the respective ablation electrode, and 3) the updated position of the electrode skin endpoint of the respective ablation electrode as the current position of the electrode skin endpoint of the respective ablation electrode to determine a final position of the electrode tumor endpoint of the respective ablation electrode and a final position of the electrode skin endpoint of the respective ablation electrode for performing a thermal ablation on the one or more tumors; and
means for outputting (614) the final position of the electrode tumor endpoint and the final position of the electrode skin endpoint of each of the plurality of ablation electrodes.

11. The apparatus or method of any one of claims 1 - 10, wherein the one or more anatomical objects further comprise one or more organs and skin of a patient.

12. The apparatus of claim 10 or 11, wherein:

the means for defining a next state of the environment comprises means for updating a joint net cumulative reward for the plurality of AI agents, wherein the joint net cumulative reward is defined based on clinical constraints; and

the means for repeating the steps of determining the one or more actions and defining the next state for a plurality of iterations comprises means for repeating the steps of determining the one or more actions and defining the next state until the joint net cumulative reward satisfies a threshold value.

13. The apparatus of any one of claims 10 - 12, wherein the means for determining one or more actions for updating the current position of the electrode tumor endpoint of a respective ablation electrode of the plurality of ablation electrodes and the current position of the electrode skin endpoint of the respective ablation electrode in the environment based on the current state using the particular AI agent comprises:
means for determining one or more discrete predefined actions using the particular AI agent implemented using a double deep Q network.

14. The apparatus of any one of claims 10 - 13, wherein the means for determining one or more actions for updating the current position of the electrode tumor endpoint of a respective ablation electrode of the plurality of ablation electrodes and the current position of the electrode skin endpoint of the respective ablation electrode in the environment based on the current state using the particular AI agent comprises:
means for determining a continuous action using the particular AI agent implemented using proximal policy optimization.

15. The apparatus of any one of claims 10 - 14, wherein the means for defining a current state of an environment based on a mask of one or more anatomical objects, a current position of an electrode tumor endpoint of each of a plurality of ablation electrodes, and a current position of an electrode skin endpoint of each of the plurality of ablation electrodes comprises:
means for defining the current state of the environment based on an ablation zone (712) of each of the plurality of ablation electrodes, wherein the ablation zones are modeled as an ellipsoid.

16. The apparatus of any one of claims 10 - 15, further comprising:
means for generating intraoperative guidance for performing a thermal ablation on the one or more tumors based on the one or more final positions.

17. A non-transitory computer readable medium storing computer program instructions, the computer program instructions when executed by a processor cause the processor to perform operations comprising the steps of any one of claims 1-9.

# FIG 1

100

Receive one or more input medical images of a patient
102

Generate a mask of one or more anatomical objects from the one or more input medical images
104

Define a current state of an environment based on the mask and one or more current positions of one or more ablation electrodes
106

Determine one or more actions for updating the one or more current positions of a respective ablation electrode of the one or more ablation electrodes in the environment based on the current state using the particular AI agent
108

Define a next state of the environment based on the mask and the one or more updated positions of the respective ablation electrode
110

Repeat the steps of determining the one or more actions and defining the next state for a plurality of iterations to iteratively update the one or more current positions of the respective ablation electrode using 1) the next state as the current state and 2) the one or more updated positions as the one or more current positions to determine one or more final positions of the respective ablation electrode for performing an ablation on the one or more tumors
112

Output the one or more final positions of each respective ablation electrode
114

# FIG 2

200

202

AI Agent(s)

208

210

state
$s_t$

reward
$r_t$

206

204

action
$a_t$

214 $r_{t+1}$

Environment

212 $s_{t+1}$

# FIG 3

300

1. Tumor, OAR, Skin masks $(m)$
2. Electrode points $(P_u, P_v)$
3. Electrode position update

$$P_v(a_v) -> P_{v+1}$$
$$S_t(m, P_u, P_{v+1}) -> S_{t+1}$$

# FIG 4

400

# FIG 5

# FIG 6

600

Receive one or more input medical images of a patient
602

↓

Generate a mask of one or more anatomical objects from the one or more input medical images
604

↓

Define a current state of an environment based on the mask, a current position of an electrode tumor endpoint of each of a plurality of ablation electrodes, and a current position of an electrode skin endpoint of each of the plurality of ablation electrodes
606

↓

Determine one or more actions for updating the current position of the electrode tumor endpoint of a respective ablation electrode and the current position of the electrode skin endpoint of the respective ablation electrode in the environment based on the current state using the particular AI agent
608

↓

Define a next state of the environment based on the mask, the updated position of the electrode tumor endpoint and the updated position of the electrode skin endpoint
610

↓

Repeat the steps of determining the one or more actions and defining the next state for a plurality of iterations using 1) the next state as the current state, 2) the updated position of the electrode tumor endpoint as the current position of the electrode tumor endpoint, and 3) the updated position of the electrode skin endpoint as the current position of the electrode skin endpoint to determine a final position of the electrode tumor endpoint and a final position of the electrode skin endpoint for performing an ablation on the one or more tumors
612

↓

Output the final position of the electrode tumor endpoint and the final position of the electrode skin endpoint of each respective ablation electrode
614

# FIG 7

<u>700</u>

ablation zone

electrode

$a_i$

712
706
702
704
708
710

# FIG 8

800

$$\mathcal{L}(\theta_i) = \sum_{i=1}^{b} [(y_i^{tot} - Q^{tot}(s, a, \theta_i))^2]$$

FIG. 9

1000

FIG. 10

# FIG 11

1102

Network interface 1106

I/O 1108

Processor 1104

Storage 1112

Memory 1110

Image Acquisition Device 1114

Europäisches
Patentamt

European
Patent Office

Office européen
des brevets

# EUROPEAN SEARCH REPORT

Application Number

EP 23 20 0125

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | Chaitanya Krishna ET AL: "Automatic planning of liver tumor thermal ablation using deep reinforcement learning", , 28 February 2022 (2022-02-28), pages 1-12, XP093094005, Retrieved from the Internet: URL:https://openreview.net/pdf?id=ehsvFoQaz-W [retrieved on 2023-10-23] * the whole document * | 1-17 | INV. G16H20/40 A61B34/10 G16H40/63 |
| L | Chaitanya Krishna ET AL: "Automatic planning of liver tumor thermal ablation using deep reinforcement learning ¦ OpenReview", openreview.net forum, 28 February 2022 (2022-02-28), XP093124563, Retrieved from the Internet: URL:https://openreview.net/forum?id=ehsvFoQaz-W [retrieved on 2024-01-26] * the whole document * | 1-17 | |

**TECHNICAL FIELDS SEARCHED (IPC)**

G16H
A61B

The present search report has been drawn up for all claims

| Place of search | Date of completion of the search | Examiner |
|---|---|---|
| Munich | 26 January 2024 | Rákossy, Z |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

................................................................
& : member of the same patent family, corresponding
document

EPO FORM 1503 03.82 (P04C01)